# EUROPEAN PATENT SPECIFICATION

(11) **EP 1 506 789 B1**
(45) Date of publication and mention of the grant of the patent: **07.10.2009**
(21) Application number: 04019266.8
(22) Date of filing: 13.08.2004
(51) Int. Cl.: A61L 17/10, A61L 27/16, A61L 27/18, A61L 15/22, A61L 31/04

(54) **Heterogeneous yarns for surgical articles**
Heterogene Fäden für chirurgische Artikel
Fils hétérogènes pour articles chirurgicaux

(30) Priority: 14.08.2003 US 494993 P
(43) Date of publication of application: 16.02.2005
(73) Proprietor: Tyco Healthcare Group LP, Norwalk, Connecticut 06856 (US)
(72) Inventor: Roby, Mark S., Killingsworth CT 06419 (US)
(74) Representative: HOFFMANN EITLE

(56) References cited:
- EP-A- 0 557 894
- EP-A- 1 293 218
- WO-A-20/04082466
- US-A- 4 695 280
- US-A- 4 834 752
- US-A- 5 366 504

## Description

### Technical Field

The present disclosure relates to yarns made of dissimilar materials. Filaments of at least two dissimilar materials are combined to form a yarn which, in turn, can be braided, woven, etc. to form a device suitable for surgical use.

### Background

Sutures intended for the repair of body tissues must meet certain requirements; they must be substantially non-toxic, capable of being readily sterilized, they must have good tensile strength and have acceptable knot-tying and knot-holding characteristics and, if the sutures are of the bio-absorbable variety, the bio-absorption of the suture must be closely controlled.

Sutures have been constructed from a wide variety of materials including surgical gut, silk, cotton, polyolefins such as polypropylene, polyamides, polyesters such as polyethylene terephthalate, polyglycolic acid, glycolide-lactide copolymer, etc. Sutures have been constructed from these materials in a monofilament form and as braided structures. For example, sutures manufactured from silk, polyamide, polyester and bio-absorbable glycolide-lactide copolymer are typically provided as multi-filament braids.

EP 1293218 describes a suture strand suitable for use as a suture or ligature comprising a plurality of braided fibers of ultrahigh molecular weight polyethylene and polyester.

Filaments have also been utilized to form other mutli-filament surgical or medical devices, such as braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices. Multi-filaments braids typically provide the advantages of enhanced pliability, and tensile strength as compared to monofilament constructions, and where utilized as sutures, posses enhanced knot security. The enhanced pliability of a multi-filament braid is caused by a lower resistance to bending of a strand of very fine filaments as opposed to one large diameter monofilament. The individual filaments must be able to bend unencumbered or unrestricted by their neighboring filaments. Any mechanism which reduces this individual fiber mobility, such as simple fiber-fiber friction, a coating which penetrates into the braid interstices, or a melted polymer matrix which adheres fibers together, could adversely affect braid pliability. It would be advantageous to combine dissimilar materials or fibers to form filament strands to enhance pliability and strength.

### Summary

The present disclosure relates to biocompatible composite surgical devices made from heterogeneous yarns. The yarns contain muttipte strands of a polyester material and multiple strands of a polyolefin material. The strands are combined in substantially parallel lengths with respect to each other to form the heterogeneous yarn. The yarns can then be braided, knitted or woven to form medical/ surgical devices, including sutures.

### Brief Description of Drawings

FIG. 1 is a cross sectional view of a heterogeneous yarn of polyester and polyolefin strands;
FIG. 2 shows a needle-suture combination in accordance with the present disclosure;
FIG. 3 is a perspective view of a portion of a split human sternum illustrating one application of the present invention for retaining the split portions together to promote healing;
FIG. 4 is an enlarged view of the suture product shown in FIG. 3 illustrating one embodiment wherein the elongated product is a flat braided member and contains at least eight reinforcing filaments extending along the length;
FIG. 5 is a view of an alternative embodiment of the suture repair product wherein the elongated member is a spiroid braided member having a generally circular cross-section containing at least one elongated reinforcing member; and
FIG. 6 is a view of another alternative embodiment of the suture repair product wherein the elongated product is a hollow braided member having a generally circular cross-section and contains at least one elongated reinforcing member extending centrally thereof along the length.

### Detailed Description

The present disclosure relates to heterogeneous yarns, which can be used in the fabrication in whole or in part of a variety of textile surgical devices, including, for example, sutures, braided tapes, gauze, wound dressings, hernia) repair meshes, vascular grafts (e.g. fabrics and/or tubes) anastomosis rings, prosthetic ligaments and tendons, growth matrices, drug delivery devices and other implantable medical devices. An implantable medical device is defined as any device which can be implanted in an animal for medical purposes. The yarns can be braided, knitted or woven to form the devices.

In accordance with the present disclosure, at least two different kinds of fibers or strands are placed in intimate contact to form a heterogeneous yarn. More particularly, the heterogeneous yarns contain strands made from a polyester and strands made from a polyolefin. Multiple strands of polyester and polyolefin are combined to form a single yarn. Multiple heterogeneous yarns thus obtained are then braided, knitted, or woven to form a multi-filament surgical/medical device, such as, for example, a multi-filament surgical suture.

In particularly useful embodiments, the polyester strands are made of polyethylene terephthalate. Polyethylene terephthalate is a thermoplastic polyester formed by esterification from ethylene glycol and terephthalic acid. Its advantageous properties include high tensile strength, high resistance to stretching under both wet and dry conditions, and good resistance to degradation by chemical bleaches and to abrasion. Polyethylene terephthalate is commercially available from DuPont Corporation, Wilmington, DE, under the trademark DACRON® .

The polyolefin strands are preferably made from a polyethylene. In particularly useful embodiments, the polyethylene is an ultra high molecular weight polyethylene. Ultra high molecular weight ("UHMW") polyethylene is a linear polymer with an average molecular weight greater than about 400,000, typically in the range of about 500,000 to about 6,000,000. UHMW polyethylene has a high tenacity and low elongation rate to provide articles with greatly increased strength and decreased elongation.

UHMW polyethylene typically exhibits a very substantial degree of crystalline orientation (95-99%) and crystalline content (60-85%). The significant strength and stability of UHMW polyethylene is normally caused by the high degree of molecular orientation. As a result, the fibers exhibit strengths from about 375 kpsi (thousands of pounds per square inch) to about 560 kpsi, and tensile moduli of about 15 msi (millions of pounds per square inch) to about 30 msi. Ultra high molecular weight polyethylene is commercially available under the trademark SPECTRA ® from Allied-Signal Technologies, Petersburg, Va., and under the trademark DYNEEMA ® from DSM High Performance Fibers, JH Heerlen, The Netherlands.

The yarn may optionally contain strands of other materials. Materials used to construct these optional strands can include a wide variety of natural and synthetic fibrous materials such as any of those previously known for the construction of sutures. Such materials include non-absorbable as well as partially and fully bio-absorbable (i.e., resorbable) natural and synthetic fiber-forming polymers, including thermoplastics. Suitable non-absorbable materials can include, for example, polyamides, polyesters such as polyethylene terephthalate, polyacrylonitrile, polyolefins such as polyethylene and polypropylene, silk, cotton, linen, etc. Carbon fibers, steel fibers and other biologically acceptable inorganic fibrous materials can also be employed. Bioabsorbable resins can include those derived from glycolic acid, glycolide, lactic acid; lactide, dioxanone, epsilon-caprolactone, trimethylene carbonate, etc., and various combinations of these and related monomers.

The strands which form the yarns can be made using any known technique, such as, for example, extrusion, molding and/or solvent casting. In a preferred embodiment, the strands can be extruded through an extruder unit of a conventional type, such as those disclosed in U.S. Pat. Nos. 6,063,105; 6,203,564; and 6,235,869. can be extruded separately and subsequently brought together into a group to form a yarn, or the strands can be extruded in a side-by-side fashion and collected together to immediately form a yarn. The number of strands used per yarn will depend on a number of factors including the desired final size of the yarn and the ultimate multifilament article being produced. For example, with respect to sutures, size is established according to United States Pharmacopoceia ("USP") standards. However, each yarn can contain at least ten strands, and often more, of at least two differing material types. The strands run parallel to each other along the length of the yarn, although twisting the strands to form a twisted yarn is also contemplated.

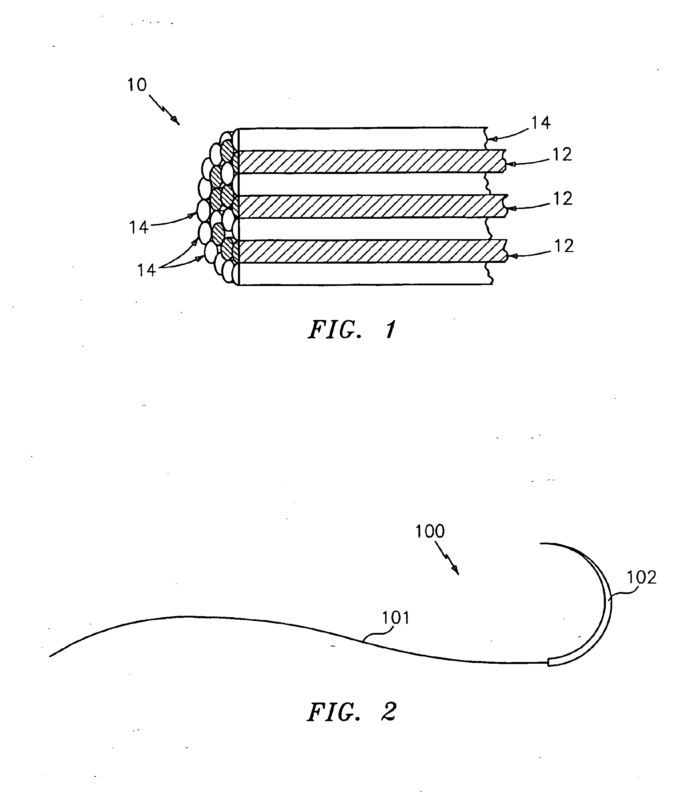

Turning now to Figure 1, a heterogeneous yarn 10 of the present disclosure is shown. The yarn 10 contains polyolefin strands 12 (such as, UHMW polyethylene) and polyester strands 14 (such as DACRON®). Once formed, a plurality of the heterogeneous yarns can then be braided, knitted, or woven together. The braiding can be done by any method known to those skilled in the art. For example, braid constructions for sutures and other medical devices are described in U.S. Pat. Nos. 5,019,093; 5,059,213; 5,133,738; 5,181,923; 5,226,912; 5,261,886; 5,306,289; 5,318,575; 5,370,031; 5,383,387; 5,662,682; 5,667,528; 6,203,564. Once the suture is constructed, it is preferably sterilized, by any means known to those skilled in the art.

Braided surgical devices made using heterogeneous yarns prepared in accordance with the disclosure can optionally be coated with one or more coating compositions to improve functional properties of the device. For example, a coating can be applied to improve surface lubricity and knot tie-down behavior. Suitable coating compositions indude but are not limited to those disclosed in U.S. Pat. Nos. 3,867,190; 3,942,532; 4,047,533; 4,452,973; 4,624,256; 4,649,920; 4,716,203; 4,826,945; and 5,569,302. The coating can be applied using any known technique such as, for example coating, dipping, spraying or other appropriate techniques.

The amount of coating composition applied to the device will vary depending upon the specific construction of the device, its size and the exact material of its construction. In general, the coating composition will constitute from about 0.5 to about 4.0 percent by weight of the coated device, or higher with a preferred range from about 1.0 percent to about 3.0 percent.

A surgical device prepared from the presently described heterogeneous yarns may also be impregnated with one or more medico-surgically useful substances, e.g., those which accelerate or beneficially modify the healing process when the suture is applied to a wound or surgical site. The medically useful or therapeutic agents can indude varying amounts of one or more optional ingredients, such as, for example, bioactive substances such as biocidal agents, antibiotics, antimicrobials, medicants, growth factors, anti-clotting agents, analgesic, anesthetics, anti-inflammatory, etc., and the like. Medicants are defined as substances which are beneficial to the animal and tend to promote the healing process. For example, a braided suture can be provided with a therapeutic agent which will be deposited at the sutured site. The therapeutic agent can be chosen for its antimicrobial properties,. capability for promoting wound repair and/or tissue growth, or for specific indications such as thrombosis. Antimicrobial agents such as broad spectrum antibiotics (gentamicin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or more biologically active materials known to achieve either or both of these objectives can also be applied to the braided suture. Such materials include any of several human Growth factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokines to enhance the immune system, and so forth.

The braided device prepared from heterogeneous yarns in accordance with this disclosure can also include, for example, biologically acceptable plasticizers, antioxidants, and colorants, which can be impregnated into the heterogeneous yarns of the device.

In addition, the yarn and/or product may be plasma treated depending upon the particular needs or intended application so as to reduce the perceived "slipperiness" of the product as desired.

The braided suture 101 prepared in accordance with this disclosure can have a needle 102 attached thereto as shown in FIG. 2, to provide a needle suture combination 100. In order to facilitate needle attachment, conventional tipping agents can be applied to the braid. Two tipped ends of the suture may be desirable for attaching a needle to each end of the suture to provide a so-called double armed suture. The needle attachment can be made by any conventional method such as crimping, swaging, etc., including those described in US Pat. Nos. 5,133,738; 5,226,912; and 5,569,302.

As noted above the heterogeneous yarns of the present disclosure can also be utilized to form other surgical or medical articles, including, braided tapes, gauze, wound dressings, hernial repair meshes, vascular grafts (e.g. fabrics and/or tubes) anastomosis rings, prosthetic ligaments and tendons, growth matrices, drug delivery devices and other implantable medical devices.

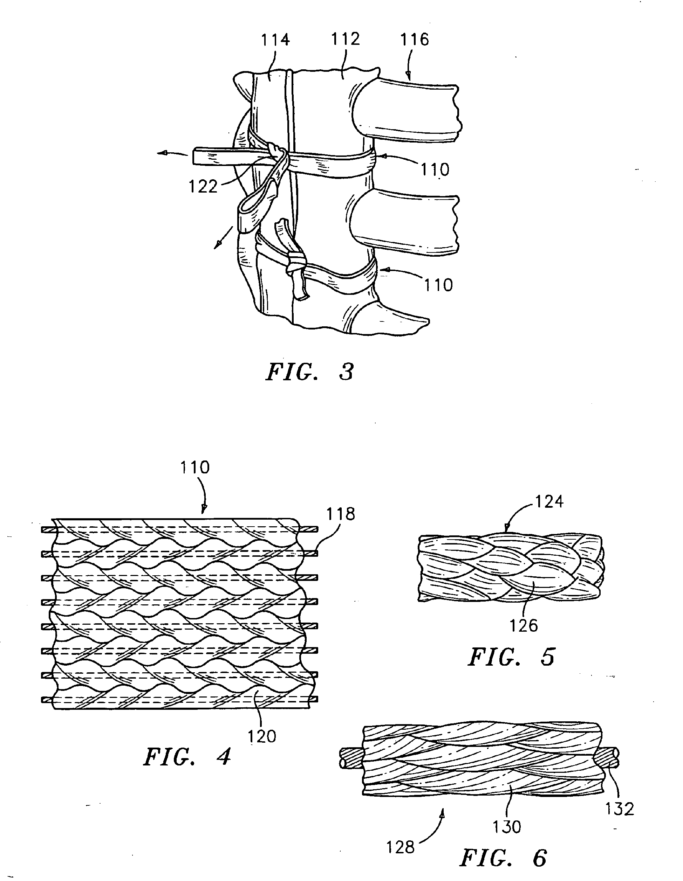

Referring initially to FIG. 3 there is illustrated a stemum closure ribbon 110 constructed according to the present invention and positioned to retain portions 112, 114 of a human sternum 116 together. The band 110 is a braided product made from the heterogeneous yarns described herein. In FIG. 4, the band 110 shown in FIG. 3 is shown in greater detail as an elongated flat braided textile product prepared as described in U.S. Patent No. 5,318,575 with the exception that heterogeneous yarns in accordance with the present disclosure are used in the construction.

Accordingly, it is possible in one application to position the reinforced structure 110 about the split portions 112, 114 of the human stemum 116 as shown in FIG. 3 whereby substantial force may be applied to the band by tying the band either by a knot 122 shown in FIG. 3, or by other techniques whereby significant force may be applied and retained to promote natural healing of the sternum portions 112, 114, e.g. mechanical connecting devices such as buckles, etc. See, for example, U.S. Pat. No. 4,813,416.

In FIG. 5, there is an alternative elongated embodiment of spiroid braided construction of generally circular cross-section and comprised of heterogeneous yarns 126 combined to form a braided rope-like construction of generally circular cross-sectional configuration. Braid constructions having a circular cross-section are described in U.S. Pat. Nos. 3,565,077 and 5,019,093. In FIG. 6 there is shown a hollow braid construction 128 having a sheath constructed of heterogeneous yarns 130 and having a core 132.

Surgical devices prepared from heterogeneous yarns in accordance with this disclosure can be packaged and sterilized in any conventional manner known to those skilled in the art.

It will be understood that various modifications may be made to the embodiments disclosed herein. For example, in any of the braided products described herein one or more of the yarns may be heterogeneous yarns in accordance with this disclosure while the remaining portions are made of absorbable or non-absorbable fibers or filaments. As one illustrative example, for braided products containing a core/sheath structure, the core may be a heterogeneous yarns in accordance with this disclosure while the sheath yarns can be made form other biocompatible fibers, including, but not necessarily, bioabsorbable fibers. Therefore the above description should not be construed as limiting, but merely as exemplications of preferred embodiments.

## Claims

1. A surgical device comprising; at least one heterogeneous yam including a plurality of polyester strands in intimate contact with a plurality of polyolefin strands, wherein the plurality of polyester strands and the plurality of the polyolefin strands are combined in parallel lengths with respect to each other to form the heterogeneous yarn.

2. A surgical device as in claim 1, wherein the polyester strands comprise polyethylene terephthalate.

3. A surgical device as in claim 1, wherein the polyolefin strands comprise polyethylene.

4. A surgical device as in claim 1, wherein the polyolefin strands comprise ultra high molecular weight polyethylene.

5. A surgical device as in claim 1, wherein the at least one heterogeneous yam further includes strands made from a material other than polyolefin or polyester.

6. A surgical device as in claim 1, wherein the polyester strands and polyolefin strands are twisted together to form the heterogeneous yarn.

7. A surgical device as in claim 1, comprising a plurality of heterogeneous yarns that are braided.

8. A surgical device as in claim 1, comprising plurality of heterogeneous yarns that are knitted.

9. A surgical device as in claim 1, comprising a plurality of heterogeneous yarns that are woven.

10. A biocompatible surgical device comprising a textile prepared from a plurality of heterogeneous yarns having strands of polyethylene terephthalate in intimate contact with strands of ultra high molecular weight polyethylene, wherein the plurality of polyethylene terephthalate strands and the plurality of the ultra high molecular weight polyethylene strands are combined in parallel lengths with respect to each other to form the heterogeneous yarn.

11. A biocompatible surgical device as in claim 10, wherein the textile comprises braided heterogeneous yarns.

12. A biocompatible surgical device as in claim 10, wherein the textile comprises knitted heterogeneous yarns.

13. A biocompatible surgical device as in claim 10, wherein the textile comprises woven heterogeneous yarns.

14. A biocompatible surgical device as in claim 10, wherein the textile is adapted for use as a device selected from the group consisting of sutures, tapes, gauze, wound dressings, hernial repair meshes, vascular grafts, anastomosis rings, prosthetic ligaments, prosthetic tendons, growth matrices and drug delivery devices.

15. A biocompatible surgical device as in claim 14, wherein the textile is a braided suture.

16. A suture comprising at least one heterogeneous yam having polyester strands in intimate contact with polyolefin strands,
wherein the plurality of polyester strands and the plurality of the polyolefin strands are combined in parallel lengths with respect to each other to form the heterogeneous yarn.

17. A suture as in claim 16, wherein the polyester strands comprise polyethylene terephthalate.

18. A suture as in claim 16, wherein the polyolefin strands comprise polyethylene.

19. A suture as in claim 16, wherein the polyolefin strands comprise ultra high molecular weight polyethylene.

20. A suture as in claim 16, wherein the heterogeneous yam is coated with a composition containing one or more biologically active materials.

## Patentansprüche

1. Chirurgische Vorrichtung, umfassend: zumindest ein heterogenes Garn mit einer Mehrzahl von Polyesterfasern in engem Kontakt mit einer Mehrzahl von Polyolefinfasern,
wobei die Mehrzahl von Polyesterfasern und die Mehrzahl von Polyolefinfasern in parallelen Streckenstücken in Bezug aufeinander kombiniert sind, um das heterogene Garn zu bilden.

2. Chirurgische Vorrichtung nach Anspruch 1, wobei die Polyesterfasern Polyethylenterephthalat umfassen.

3. Chirurgische Vorrichtung nach Anspruch 1, wobei die Polyolefinfasern Polyethylen umfassen.

4. Chirurgische Vorrichtung nach Anspruch 1, wobei die Polyolefinfasern ultrahochmolekulares Polyethylen umfassen.

5. Chirurgische Vorrichtung nach Anspruch 1, wobei das zumindest eine heterogene Garn ferner Fasern enthält, die aus einem anderen Material als Polyolefin oder Polyester gemacht sind.

6. Chirurgische Vorrichtung nach Anspruch 1, wobei die Polyesterfasern und Polyolefinfasern zusammen verdrillt sind, um das heterogene Garn zu bilden.

7. Chirurgische Vorrichtung nach Anspruch 1, umfassend eine Mehrzahl von heterogenen Garnen, die geflochten sind.

8. Chirurgische Vorrichtung nach Anspruch 1, umfassend mehrere heterogene Garne, die gestrickt sind.

9. Chirurgische Vorrichtung nach Anspruch 1, umfassend eine Mehrzahl von heterogenen Garnen, die gewoben sind.

10. Biokompatible chirurgische Vorrichtung, umfassend ein Textil, das aus einer Mehrzahl von heterogenen Garnen präpariert ist, die Fasern aus Polyethylenterephthalat in engem Kontakt mit Fasern aus ultrahochmolekularem Polyethylen aufweisen,
wobei die Mehrzahl von Polyethylenterephthalatfasern und die Mehrzahl der ultrahochmolekularen Polyethylenfasern in parallelen Streckenstücken in Bezug aufeinander kombiniert sind, um das heterogene Garn zu bilden.

11. Biokompatible chirurgische Vorrichtung nach Anspruch 10, wobei das Textil geflochtene heterogene Garne aufweist.

12. Biokompatible chirurgische Vorrichtung nach Anspruch 10, wobei das Textil gestrickte heterogene Garne aufweist.

13. Biokompatible chirurgische Vorrichtung nach Anspruch 10, wobei das Textil gewobene heterogene Garne aufweist.

14. Biokompatible chirurgische Vorrichtung nach Anspruch 10, wobei das Textil für eine Verwendung als eine Vorrichtung angepasst ist, die aus der Gruppe ausgewählt ist, die aus Nähten, Bändern, Gaze, Wundverbänden, Bruchoperationsnetzen, Gefäßprothesen, Anastomoseringen, Ligamentprothesen, Sehnenprothesen, Wuchsmatrizen und Arzneimittelzuführungsvorrichtungen besteht.

15. Biokompatible chirurgische Vorrichtung nach Anspruch 14, wobei das Textil ein geflochtener Nähfaden ist.

16. Nähfaden, umfassend zumindest ein heterogenes Garn mit Polyesterfasern in engem Kontakt mit Polyolefinfasern,
wobei die Mehrzahl von Polyesterfasern und die Mehrzahl von Polyolefinfasern in parallelen Streckenstücken in Bezug aufeinander kombiniert sind, um das heterogene Garn zu bilden.

17. Nähfaden nach Anspruch 16, wobei die Polyesterfasern Polyethylenterephthalat umfassen.

18. Nähfaden nach Anspruch 16, wobei die Polyolefinfasern Polyethylen umfassen.

19. Nähfaden nach Anspruch 16, wobei die Polyolefinfasern ultrahochmolekulares Polyethylen umfassen.

20. Nähfaden nach Anspruch 16, wobei das heterogene Garn mit einer Verbindung bedeckt ist, die ein oder mehrere biologisch aktive Materialien enthält.

## Revendications

1. Dispositif chirurgical comprenant: au moins un fil hétérogène comprenant une pluralité de paquets de fibres en polyester en contact intime avec une pluralité de paquets de fibres en polyoléfine,
dans lequel la pluralité de paquets de fibres en polyester et la pluralité de paquets de fibres en polyoléfine sont combinés en des longueurs parallèles les uns par rapport aux autres pour former un fil hétérogène.

2. Dispositif chirurgical selon la revendication 1, dans lequel les paquets de fibres en polyester comprennent du polyéthylène téréphtalate.

3. Dispositif chirurgical selon la revendication 1, dans lequel les paquets de fibres en polyoléfine comprennent du polyéthylène.

4. Dispositif chirurgical selon la revendication 1, dans lequel les paquets de fibres en polyoléfine comprennent du polyéthylène d'un poids moléculaire ultra-élevé.

5. Dispositif chirurgical selon la revendication 1, dans lequel le au moins un fil hétérogène comprend en outre des paquets de fibres réalisés en un matériau autre que la polyoléfine ou le polyester.

6. Dispositif chirurgical selon la revendication 1, dans lequel les paquets de fibres en polyester et les paquets de fibres en polyoléfine sont tordus ensemble pour former le fil hétérogène.

7. Dispositif chirurgical selon la revendication 1, comprenant une pluralité de fils hétérogènes qui sont tressés.

8. Dispositif chirurgical selon la revendication 1, comprenant une pluralité de fils hétérogènes qui sont tricotés.

9. Dispositif chirurgical selon la revendication 1, comprenant une pluralité de fils hétérogènes qui sont tissés.

10. Dispositif chirurgical biocompatible comprenant un textile préparé à partir d'une pluralité de fils hétérogènes ayant des paquets de fibres en polyéthylène téréphtalate en contact intime avec des paquets de fibres de polyéthylène d'un poids moléculaire ultra-élevé,
dans lequel la pluralité de paquets de fibres en polyéthylène téréphtalate et la pluralité de paquets de fibres en polyéthylène d'un poids moléculaire ultra-élevé sont combinés en des longueurs parallèles les uns par rapport aux autres pour former le fil hétérogène.

11. Dispositif chirurgical biocompatible selon la revendication 10, dans lequel le textile comprend des fils hétérogènes tressés.

12. Dispositif chirurgical biocompatible selon la revendication 10, dans lequel le textile comprend des fils hétérogènes tricotés.

13. Dispositif chirurgical biocompatible selon la revendication 10, dans lequel le textile comprend des fils hétérogènes tissés.

14. Dispositif chirurgical biocompatible selon la revendication 10, dans lequel le textile est conçu pour utilisation comme un dispositif sélectionné dans le groupe consistant en sutures, bandes, gaz, pansements, mailles de réparation d'hernies, greffes vasculaires, anneaux d'anastomose, ligaments prothétiques, tendons prothétiques, matrice de croissance et dispositifs de libération de médicaments.

15. Dispositif chirurgical biocompatible selon la revendication 14, dans lequel le textile est une suture tressée.

16. Suture comprenant au moins un fil hétérogène ayant des paquets de fibres en polyester en contact intime avec des paquets de fibres en polyoléfine, la pluralité de paquets de fibres en polyester et la pluralité de paquets de fibres en polyoléfine étant combinés en des longueurs parallèles les uns par rapport aux autres pour former le fil hétérogène.

17. Suture selon la revendication 16, où les paquets de fils en polyester comprennent du polyéthylène téréphtalate.

18. Suture selon la revendication 16, où les paquets de fibres en polyoléfine comprennent du polyéthylène.

19. Suture selon la revendication 16, où les paquets de fibres en polyoléfine comprennent du polyéthylène d'un poids moléculaire ultra-élevé.

20. Suture selon la revendication 16, où le fil hétérogène est revêtu d'une composition contenant un ou plusieurs matériaux biologiquement actifs.
